# EUROPÄISCHE PATENTSCHRIFT

(11) **EP 1 516 865 B1**
(45) Veröffentlichungstag und Bekanntmachung des Hinweises auf die Patenterteilung: **21.11.2007**
(21) Anmeldenummer: 04021663.2
(22) Anmeldetag: 11.09.2004
(51) Int. Cl.: C07C 69/003, C08K 5/11, C10M 105/36

(54) **Carbonsäureester auf Basis von Limonanalkohol (3-(4'-Methylcyclohexyl)butanol) mit niedrigem Stockpunkt**
3-(4'-methylcyclohexyl) butanol based carboxylic acid esters having low pour point
Esters d' acides carboxyliques à base de 3-(4'-méthylcyclohexyl) butanol à bas point d' écoulement

(30) Priorität: 20.09.2003 DE 10343623
(43) Veröffentlichungstag der Anmeldung: 23.03.2005
(73) Patentinhaber: OXEA Deutschland GmbH, 46147 Oberhausen (DE)
(72) Erfinder: Lappe, Peter, Dr., 46539 Dinslaken (DE); Balzarek, Christoph, Dr., 47051 Duisburg (DE)

(56) Entgegenhaltungen:
- GB-A- 880 961
- US-A- 2 889 354
- KIRK-OTHMER: "Encyclopedia of Chemical Technology, 3rd Edition, Vol. 14" 1981, JOHN WILEY & SONS * Seite 488 - Seite 489 *

## Beschreibung

Die vorliegende Erfindung betrifft neue Carbonsäureester auf Basis von Limonanalkohol, Verfahren zu ihrer Herstellung sowie ihre Verwendung.

Carbonsäureester finden in großem Umfang und in vielfältiger Weise Anwendung in der Technik, wie zum Beispiel als Schmiermittel, Weichmacher und Riechstoffe. In der Industrie werden zahlreiche unterschiedliche Ester eingesetzt, ausgehend von einfachen Carbonsäureestern aus Monocarbonsäuren und Monoalkoholen bis hin zu komplexen Esterölen aus Mischungen von Mono- und Dicarbonsäuren mit mono- und polyfunktionellen Alkoholen. Durch die Auswahl geeigneter Ausgangsprodukte lassen sich die physikalischen Stoffeigenschaften, wie z.B. Siedepunkt oder Viskosität gezielt einstellen, und den chemischen Eigenschaften, wie z.B. der Hydrolysestabilität oder der Stabilität gegenüber einem oxidativen Abbau Rechnung tragen. Auch auf die Lösung konkreter anwendungstechnischer Probleme können Carbonsäureester gezielt zugeschnitten werden.

Ausführliche Übersichten über den Einsatz von Carbonsäureestern finden sich z.B. in Ullmann's Encyclopedia of Industrial Chemistry, 5. Auflage, 1988, VCH; Vol. A11, Seiten 191-193, Vol. A15, Seiten 438-440, Vol. A20, Seiten 439-458; Synthetic Lubricants and High-Performance Functional Fluids, Marcel Dekker Inc., 1999, 2. Auflage, Seiten 63-102; Common Fragrance and Flavor Materials, Wiley-VCH 2001.

Die Verwendung von Carbonsäureestern als Schmierstoffe besitzt eine große technische Bedeutung. Der Begriff "Schmierstoffe" umfasst streng genommen nur Produkte, die für die Schmierung gleitender oder rollender Elemente verwendet werden. Die in der Technik in zahlreichen Anwendungen eingesetzten Schmierstoffe bestehen überwiegend aus Mineralölen oder voll- oder teilsynthetischen Produkten. Schmierstoffe auf Basis von Mineralölen sind sehr vielseitig anwendbar. Sie dienen nicht nur zur Schmierung und Kraftübertragung bei hohen und tiefen Temperaturen, sondern auch zur Wärmeübertragung und Isolierung. Für Anforderungen, die Mineralölprodukte nur unvollständig erfüllen, können synthetische Flüssigkeiten mit schmierölartigem Charakter zu technisch besseren Lösungen führen. Synthetische Grundöle werden aus weitgehend einheitlichen Substanzen unter kontrollierten Bedingungen hergestellt und können unterschiedlichen chemischen Verbindungsklassen angehören.

Eine besonders wichtige Verbindungsklasse stellen die Esteröle dar. Diese werden in großem Stil beispielsweise im Flugverkehr als Turbinenmotoren- und Instrumentenöle, als Fette oder Waffenöle verwendet. Diese Esteröle werden durch die Umsetzung von Säuren oder Säureanhydride, insbesondere von Mono- oder Dicarbonsäuren und Alkoholen, insbesondere von Mono-, Di-, Tri- oder Tetra-Alkoholen hergestellt.

Technisch wichtige Ausgangsprodukte für Ester sind z.B. aliphatische Monocarbonsäuren mit 5-10 Kohlenstoffatomen. Als Dicarbonsäuren stehen Adipinsäure, Azelainsäure oder Sebacinsäure in technischen Mengen zur Verfügung. Als Alkohole kommen neben den aliphatischen Alkoholen wie 2-Ethylhexanol vor allem mehrwertige Alkohole wie Ethylenglykol und seine Oligomeren Di-, Tri- und Tetraethylenglykol, Propylenglykol und seine Oligomeren, Propandiol-1.3, Butandiol-1.4, Hexandiol-1.6, Neopentylglykol, Trimethylolpropan, Glycerin und Pentaerythrit zum Einsatz.

Die Entwicklung moderner Schmierstoffe und deren richtige Anwendung sind von erheblicher wirtschaftlicher Tragweite. Optimal der jeweiligen Aufgabe angepasste Schmierstoffe bringen durch Energieeinsparung, Verschleißminderung, verminderte Wartungszeiten und verlängerte Überholungsintervalle erhebliche Einsparungen. Daher besteht trotz der bereits zahlreich im Alltag und in der Technik eingeführten Produkte ein Bedarf an neuen Schmierstoffen mit verbesserten Eigenschaften. Insbesondere das Viskositäts- und Stockpunktverhalten von Esterverbindungen ist für viele Anwendungen bei niedrigen Temperaturen eine kritische Eigenschaft. Die sogenannten kinematischen Viskositätswerte werden gemäß DIN 51562/ASTM D445 gemessen. Das Kälteverhalten von Estern wird über den Stockpunkt oder den Pourpunkt beschrieben. Seine Messung erfolgt im allgemeinen gemäß ASTM D97.

Die Verwendung von Carbonsäureestern als Weichmacher besitzt ebenfalls eine große wirtschaftliche Bedeutung. Weichmacher finden in großem Umfang und in vielfältiger Weise Anwendung in Kunststoffen, Beschichtungsmitteln, Dichtungsmassen, Kautschuk- und Gummiartikeln. Sie treten mit hochpolymeren thermoplastischen Stoffen, ohne chemisch zu reagieren, vorzugsweise durch ihre Löse- und Quellvermögen in physikalische Wechselwirkung. Hierdurch bildet sich ein homogenes System aus, dessen thermoplastischer Bereich gegenüber den ursprünglichen Polymeren zu niederen Temperaturen verschoben ist, u.a. mit dem Ergebnis, dass seine mechanischen Eigenschaften optimiert, z.B. Formveränderungsvermögen, Elastizität, Festigkeit erhöht werden und die Härte verringert wird. Um Weichmachern möglichst weite Anwendungsgebiete zu eröffnen, müssen sie eine Reihe von Kriterien erfüllen. Im Idealfall sollten sie geruchslos, farblos, licht-, kälte- und wärmebeständig sein. Überdies erwartet man, dass sie unempfindlich gegenüber Wasser, schwer brennbar und wenig flüchtig sind und die Gesundheit nicht schädigen. Weiterhin soll die Herstellung der Weichmacher einfach sein und, um ökologischen Anforderungen zu genügen, unter Vermeidung von Abfallstoffen, wie nicht weiterverwertbaren Nebenprodukten und Schadstoffe enthaltenden Abwässern, erfolgen.

Zu den wichtigsten Weichmachern gehören die Ester von Di- und Polycarbonsäuren mit Weichmacheralkoholen, d.h. unverzweigten oder verzweigten primären Alkoholen mit etwa 6 bis 20 Kohlenstoffatomen, die als individuelle Verbindungen oder auch als Gemische eingesetzt werden. Als grossvolumige Esterweichmacher werden insbesondere Phthalsäurester zur Weichmachung von PVC verwendet.

Eine spezielle Klasse von Esterweichmachern, die auch mit der Bezeichnung G-Ester abgekürzt werden, enthält als Alkoholkomponente Diole bzw. Etherdiole, nämlich Ethylenglykol, Diethylenglykol, Triethylenglykol und Tetraethylenglykol.

Wie schon bei den Schmierstoffen ist die Entwicklung von modernen Weichmachern, die für die jeweilige anwendungstechnische Fragestellung zugeschnitten sind, von erheblicher wirtschaftlicher Bedeutung. Der Bedarf an optimal der jeweiligen Aufgabe angepassten Weichmachern mit verbesserten Eigenschaften ist trotz der zahlreichen, am Markt eingeführten Produkte von großem Interesse.

Neben den Bemühungen zur gezielten Einstellung der anwendungstechnischen Eigenschaften von Esterverbindungen werden seit geraumer Zeit Anstrengungen unternommen, die Ausgangsstoffe von Esterverbindungen, d.h. die Säure- und/oder die Alkoholkomponente, aus nachwachsenden Rohstoffen zu gewinnen und so dieser Rohstoffbasis gegenüber der Erdölbasis ein stärkeres Gewicht zu geben. Beispiele für solche Esterverbindungen und ihre Verwendung als Schmierstoffe sind die auf Ölsäuren basierenden Neopentyl-, Trimethylolpropan- oder Pentaerythritester, wie zum Beispiel die Produkte Edenor^{®} PDO oder Edenor^{®} 2742 der Firma Cognis.

Aufgabe der Erfindung ist es daher, Carbonsäurester bereitzustellen, die mit besonders gutem Erfolg als Schmierstoffe oder Weichmacher eingesetzt werden können und die auf nachwachsenden Rohstoffen basieren. Ebenfalls liegt der Erfindung die Aufgabe zugrunde, ein Verfahren bereitzustellen, das die Herstellung solcher Carbonsäureester aus leicht zugänglichen, in ausreichender Menge preisgünstig zur Verfügung stehenden Ausgangsprodukten auf Basis nachwachsender Rohstoffe erlaubt. Besonderer Wert wird in diesem Zusammenhang darauf gelegt, dass der Veresterungsprozess mit einfachen technischen Mitteln realisiert werden kann und keine aufwendigen oder spezialisierte Apparaturen erfordert.

Die vorliegende Erfindung betrifft Carbonsäureester der allgemeinen Formel 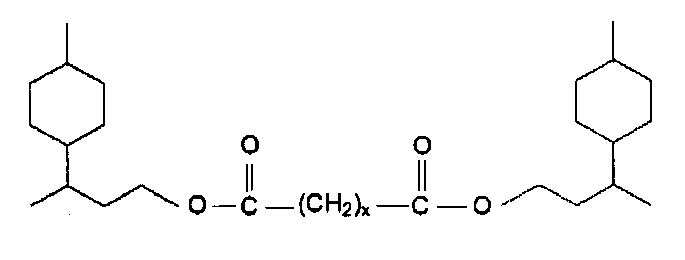 in der x = 1 - 10 bedeutet.

Als Alkoholkomponente in den neuenEsterverbindungen verwendet man Limonanalkohol [3-(4'-Methylcyclohexyl)butanol]. Die Herstellung von Limonanalkohol als Alkoholkomponente erfolgt durch Hydroformylierung von Limonen. Limonen steht in großen Mengen preisgünstig zur Verfügung und wird aus etherischen Ölen, wie zum Beispiel aus Orangenöl oder Fichtennadelöl gewonnen. Limonen findet Anwendung zur Parfümerierung von Waschmitteln sowie in der Farben- und Lackindustrie. Die unter Rh-Katalyse durchgeführte Hydroformylierung von Limonen führt in sehr hohen Ausbeuten zum Limonenaldehyd [3-(4'-Methyl-3-cyclohexen-1-yl)butyraldehyd]. Die Umsetzung mit Synthesegas erfolgt im allgemeinen in einem üblichen organischen Lösungsmittel, wie Cyclohexan, Toluol oder n-Hexan bei Temperaturen von 80 bis 150°C und Drücken von 10 bis 30 MPa in Gegenwart bekannter organischer Phosphor(III)-Verbindungen, wie zum Beispiel Triphenylphosphin, als Liganden. Limonenaldehyd wird durch destillative Aufarbeitung aus dem rohen Hydroformylierungsprodukt gewonnen und anschließend in Gegenwart üblicher Hydrierkatalysatoren mit Wasserstoff bei erhöhtem Druck und erhöhter Temperatur zum Limonanalkohol umgesetzt. Es lassen sich die in der Technik gängigen Hydrierkatalysatoren, wie zum Beispiel trägerhaltige oder trägerfreie Metallkatalysatoren, die beispielsweise Nickel, Palladium oder Kupfer als katalytisch aktives Metall enthalten, einsetzen. Darüber hinaus können gegebenenfalls Promotoren, wie Zirkon oder Mangan zugegen sein. Übliche Trägermaterialien sind Siliziumdioxid oder Aluminiumoxid.

Die Hydrierreaktion wird unter üblichen Temperaturbedingungen in einem Bereich von 70 bis 150°C und gängigen Druckbedingungen in einem Bereich von 2 bis 30 MPa durchgeführt. Die Hydrierreaktion verläuft in hohen Ausbeuten. Damit steht Limonanalkohol nach einem technisch einfachen Verfahren kostengünstig für die Herstellung neuer Carbonsäureester zur Verfügung.

Als Dicarbonsäuren kommen vor allem die aliphatischen Vertreter - Malonsäure (x=1), Bernsteinsäure (x = 2), Glutarsäure (x = 3), Adipinsäure (x = 4), Azelainsäure (x = 7), Sebacinsäure (x = 8) und 1,12-Dodecandisäure (x = 10) zur Anwendung. Diese einfachen Vertreter der aliphatischen Dicarbonsäuren sind in technischem Maßstab verfügbar oder können nach bekannten Verfahren hergestellt werden.

Die unmittelbare Veresterung von Alkoholen mit Carbonsäuren gehört zu den Grundoperationen der organischen Chemie. Um die Reaktionsgeschwindigkeit zu erhöhen, führt man die Umsetzung üblicherweise in Gegenwart von Katalysatoren durch. Der Einsatz eines der Reaktanten im Überschuß und/oder die Abtrennung des im Verlauf der Reaktion gebildeten Wassers stellt sicher, dass das Gleichgewicht entsprechend dem Massenwirkungsgesetz zur Seite des Reaktionsproduktes, also des Esters, verschoben wird, d.h. hohe Ausbeuten erzielt werden.

Für die Abtrennung des bei der Esterbildung gebildeten Reaktionswassers sind verschiedene Verfahren bekannt. Anwendung findet vorzugsweise die Azeotropdestillation in Gegenwart eines mit Wasser nicht mischbaren Lösemittels, das Erhitzen des Reaktionsgemisches unter Durchleiten eines inerten Gases, die Umsetzung der Ausgangsstoffe Alkohol und Carbonsäure unter Vakuum oder in Gegenwart eines Trocknungsmittels.

Insbesondere die Wasserentfernung durch Azeotropdestillation hat sich für die Einstellung des Gleichgewichts bei der Herstellung von Estern bewährt. Üblicherweise setzt man als Azeotropbildner organische Lösemittel ein, die im technischen Maßstab preiswert zur Verfügung stehen. Geeignet sind aber auch alle anderen organischen Substanzen mit entsprechendem Siedepunkt, die mit Wasser Azeotrope bilden. Beispiele für eingesetzte Schleppmittel sind Hexan, 1-Hexen, Cyclohexan, Toluol, Benzol.

Die zur vollständigen Abtrennung des Wassers erforderliche Menge Schleppmittel lässt sich aus der entsprechend der Stöchiometrie der Veresterungsreaktion berechneten Wasserbildung und aus der Zusammensetzung des binären Azeotrops in einfacher Weise ermitteln. Es hat sich bewährt, das Schleppmittel im Überschuss einzusetzen, zweckmäßig in einem Anteil, der 50 bis 200 Gew.-% über der theoretisch berechneten Menge liegt. Durch Auffangen und Auftrennen des abdestillierten Schleppmittel-/Wassergemischs lässt sich in einfacher Weise der Fortgang der Reaktion verfolgen. Das aus dem Azeotrop abgeschiedene Schleppmittel kann unmittelbar, d.h. ohne die Zwischenschaltung einer Reinigungsstufe, in die Reaktion zurückgeführt werden.

Die Reaktion von Limonanalkohol und Carbonsäure kann ohne Einsatz eines Katalysators durchgeführt werden. Diese Variante der Umsetzung hat den Vorteil, dass man vermeidet, dem Reaktionsgemisch Fremdstoffe zuzuführen, die zu einer unerwünschten Verunreinigung des Esters führen kann. Allerdings muss man dann im allgemeinen höhere Reaktionstemperaturen einhalten, weil nur so gewährleistet ist, dass die Umsetzung mit ausreichender, d.h. wirtschaftlich vertretbarer Geschwindigkeit anläuft. Zu beachten ist in diesem Zusammenhang, dass die Steigerung der Reaktionstemperatur zu einer thermischen Schädigung des Esters führen kann. Daher lässt sich nicht immer die Verwendung eines Katalysators, der die Umsetzung erleichtert und die Reaktionsgeschwindigkeit erhöht, vermeiden. Häufig kann der Katalysator ein Überschuß der Säure sein, die gleichzeitig Reaktionskomponente des Limonanalkohols ist. Im übrigen sind die üblichen Veresterungskatalysatoren zur Beeinflussung der Reaktionsgeschwindigkeit geeignet, wie Mineralsäuren, wie Schwefelsäure, Phosphorsäure, Polyphosphorsäure oder deren saure Salze, Trialkyl- oder Triarylphosphate, Ameisensäure, Methansulfonsäure oder p-Toluolsulfonsäure.

Die Menge des eingesetzten Katalysators kann sich über einen weiten Bereich erstrecken. Es können von 1 bis zu 10 Mol-% Katalysator, bezogen auf eingesetzte Dicarbonsäure, verwendet werden. Da größere Katalysatormengen jedoch kaum Vorteile ergeben, beträgt die Katalysatorkonzentration üblicherweise 2 bis 8, vorzugsweise 3 bis 6 Mol-%, jeweils bezogen auf eingesetzte Dicarbonsäure. Zweckmäßig entscheidet man gegebenenfalls durch Vorversuche für jeden Einzelfall, ob ohne Katalysator bei höherer Temperatur oder mit Katalysator bei niedriger Temperatur zu arbeiten ist.

Die Veresterung kann in stöchiometrischen Mengen an Limonanalkohol und Säure vorgenommen werden. Vorzugsweise setzt man jedoch Limonanalkohol im Überschuss ein, um in endlicher Zeit eine möglichst vollständige Umsetzung zu erreichen.

Die Reaktion zwischen Limonanalkohol und der Säure setzt in Abhängigkeit von den Einsatzmaterialien im Bereich von etwa 80 bis 110°C ein. Sie kann bei Temperaturen von 160 bis 200°C bei Normaldruck zu Ende geführt werden. Bei diesen Temperaturangaben handelt es sich um Richtwerte, die zweckmäßig eingehalten werden. Niedrigere Temperaturen können z.B. ausreichen, wenn im speziellen Fall eine genügend hohe Reaktionsgeschwindigkeit erzielt wird oder nur Teilumsätze angestrebt werden. Höhere Temperaturen sind möglich, wenn das Auftreten von Zersetzungsprodukten, die u.a. farbschädigend wirken, auszuschließen ist. Die Anwendung von vermindertem oder erhöhtem Druck ist nicht ausgeschlossen, sie wird sich jedoch auf Sonderfälle beschränken.

Das nach beendeter Umsetzung anfallende Reaktionsgemisch enthält neben dem Ester als erwünschtem Reaktionsprodukt gegebenenfalls nicht umgesetzte Einsatzstoffe, insbesondere noch überschüssigen Limonanalkohol, sofern mit einem Alkoholüberschuss gearbeitet wurde. Zur Aufarbeitung wird der Reaktoraustrag nach konventionellen Verfahren vom Katalysator befreit. Liegt der Katalysator als Feststoff vor, z.B. in Form eines Hydrogensulfats, filtriert man das Produkt in üblichen Filterapparaten bei normaler Temperatur oder bei Temperaturen bis 150°C. Die Filtration kann durch gängige Filtrierhilfsmittel, wie Aluminiumoxid, Kieselgel, Kieselgur, Aktivkohle unterstützt werden. Anschließend destilliert man überschüssige und nichtumgesetzte Ausgangsstoffe ab. Um letzte Reste acider Bestandteile zu entfernen, kann man auch noch eine Behandlung mit einem alkalischen Reagenz, z.B. wässriger Soda- oder Natriumhydroxidlösung vorsehen. Nach Phasentrennung wird der Ester getrocknet, beispielsweise, indem man ein inertes Gas durch das Produkt leitet oder Vakuum anlegt. Sofern der Katalysator im Reaktionsgemisch gelöst vorliegt, wie Schwefelsäure oder para-Toluolsulfonsäure, destilliert man gegebenenfalls nach vorangegangener Filtration, zunächst noch vorhandene Ausgangsstoffe ab, und behandelt darauf hin mit einem alkalischen Reagenz. Gegebenenfalls unterzieht man das Produkt einer Wasserdampfdestillation vor dem abschließenden Trocknungsschritt.

Falls es der vorgesehene Verwendungszweck erfordert, können sich der Isolierung des Esters noch weitere Reinigungsschritte anschließen, beispielsweise eine fraktionierte Destillation im Vakuum.

Die Veresterungsreaktion kann absatzweise oder auch kontinuierlich in den für die chemische Technik typischen Reaktionsapparaten durchgeführt werden. Bewährt haben sich Rührkessel, die mit einer Heizvorrichtung sowie einer Einrichtung zum Zuführen des Azeotropbildners ausgestattet sind.

Die erfindungsgemäßen Esterverbindungen eignen sich aufgrund ihres hervorragenden Viskositäts- und Kälteverhaltens ausgezeichnet als Schmierstoffe, die bei niedrigen Temperaturen eingesetzt werden aber die dennoch ein hohes Molekulargewicht aufweisen. Zudem sind die erfindungsgemäßen Ester aus dem nachwachsenden Rohstoff Limonen leicht zugänglich. Ebenfalls sind sie als Weichmacher für viele gängigen hochpolymeren thermoplastischen Kunststoffe geeignet.

Die folgenden Beispielen dienen zur Erläuterung der Erfindung, ohne sie darauf zu beschränken.

### Beispiel 1

### Herstellung von Di-(3-(4'-Methylcyclohexyl)butyl)-malonsäureester

72,8 g Malonsäure (0,7 mol), 279,3 g (1,6 mol) 3-(4'-Methylcyclohearyl)butanol, 6,6 g p-Toluolsulfonsäure Monohydrat (0,035 mol) und 50 g Toluol werden in einem 11-Dreihalskolben mit Rührer, Innenthermometer und Wasserabscheider vorgelegt und zum Rückfluss erhitzt. Innerhalb von 60 Minuten werden 26,2 g Wasser ausgekreist. Die Temperatur des Reaktionsgemisches erhöht sich zum Ende der Reaktion auf 176°C. Das Reaktionsgemisch wird auf Raumtemperatur abgekühlt und mit 18,6 g wässriger Natronlauge (1 Gew.-%) sowie 64,3 g Wasser versetzt. Nach Phasentrennung wird die organische Phase zweimal mit insgesamt 227,2 g Wasser gewaschen. Nach zweimaliger Phasentrennung wird die organische Phase (394,5 g) bei einer Kopftemperatur von 208°C und einem Druck von 100 Pa fraktioniert destilliert. Der Ester (221,8 g) wird in einer Reinheit von 92,6 % isoliert. Dies entspricht einer Ausbeute von 77,1 % der Theorie.

### Beispiel 2

### Herstellung von Di-(3-(4'-Methylcyclohexyl)butyl)-bernsteinsäureester

82,7 g Bernsteinsäure (0,7 mol), 279,3 g (1,6 mol) 3-(4'-Methylcyclohexyl)butanol, 6,6 g p-Toluolsulfonsäure Monohydrat (0,035 mol) und 50 g Toluol werden in einem 11-Dreihalskolben mit Rührer, Innenthermometer und Wasserabscheider vorgelegt und zum Rückfluss erhitzt. Innerhalb von 60 Minuten werden 26,8 g Wasser ausgekreist. Die Temperatur des Reaktionsgemisches erhöht sich zum Ende der Reaktion auf 178°C. Das Reaktionsgemisch wird auf Raumtemperatur abgekühlt und mit 15,8 g wässriger Natronlauge (1 Gew.-%) sowie 85,3 g Wasser versetzt. Nach Phasentrennung wird die organische Phase zweimal mit insgesamt 224,4 g Wasser gewaschen. Nach zweimaliger Phasentrennung wird die organische Phase (430,5 g) bei einer Kopftemperatur von 205°C und einem Druck von 100 Pa fraktioniert destilliert. Der Ester (252,0 g) wird in einer Reinheit von 91,0 % isoliert. Dies entspricht einer Ausbeute von 85,7 % der Theorie.

### Beispiel 3

### Herstellung von Di-(3-(4'-Methylcyclohexyl)butyl)-glutarsäureester

92,5 g Glutarsäure (0,7 mol), 279,3 g (1,6 mol) 3-(4'-Methylcyclohexyl)butanol, 6,6 g p-Toluolsulfonsäure Monohydrat (0,035 mol) und 50 g Toluol werden in einem 11-Dreihalskolben mit Rührer, Innenthermometer und Wasserabscheider vorgelegt und zum Rückfluss erhitzt. Innerhalb von 60 Minuten werden 26,5 g Wasser ausgekreist. Die Temperatur des Reaktionsgemisches erhöht sich zum Ende der Reaktion auf 176°C. Das Reaktionsgemisch wird auf Raumtemperatur abgekühlt und mit 11,0 g wässriger Natronlauge (1 Gew.-%) sowie 74,1 g Wasser versetzt. Nach Phasentrennung wird die organische Phase zweimal mit insgesamt 268,4 g Wasser gewaschen. Nach zweimaliger Phasentrennung wird die organische Phase (384,5 g) bei einer Kopftemperatur von 207°C und einem Druck von 100 Pa fraktioniert destilliert. Der Ester (244,0 g) wird in einer Reinheit von 90,9 % isoliert. Dies entspricht einer Ausbeute von 80,0 % der Theorie.

### Beispiel 4

### Herstellung von Di-(3-(4'-Methylcyclohexyl)butyl)-adipinsäureester

102,3 g Adipinsäure (0,7 mol), 279,3 g (1,6 mol) 3-(4'-Methylcyclohexyl)butanol, 6,6 g p-Toluolsulfonsäure Monohydrat (0,035 mol) und 50 g Toluol werden in einem 11-Dreihalskolben mit Rührer, Innenthermometer und Wasserabscheider vorgelegt und zum Rückfluss erhitzt. Innerhalb von 60 Minuten werden 27,2 g Wasser ausgekreist. Die Temperatur des Reaktionsgemisches erhöht sich zum Ende der Reaktion auf 184°C. Das Reaktionsgemisch wird auf Raumtemperatur abgekühlt und mit 16,1 g wässriger Natronlauge (1 Gew.-%) sowie 106,4 g Wasser versetzt. Nach Phasentrennung wird die organische Phase zweimal mit insgesamt 245,3 g Wasser gewaschen. Nach zweimaliger Phasentrennung wird die organische Phase (410,5 g) bei einer Kopftemperatur von 210°C und einem Druck von 100 Pa fraktioniert destilliert. Der Ester (290,0 g) wird in einer Reinheit von 91,7 % isoliert. Dies entspricht einer Ausbeute von 91,4 % der Theorie.

### Beispiel 5

### Herstellung von Di-(3-(4'-Methylcyclohexyl)butyl)-sebacinsäureester

161,8 g Sebacinsäure (0,8 mol), 290,2 g (1,6 mol) 3-(4'-Methylcyclohexyl)butanol, 7,6 g p-Toluolsulfonsäure Monohydrat (0,040 mol) und 50 g Toluol werden in einem 11-Dreihalskolben mit Rührer, Innenthermometer und Wasserabscheider vorgelegt und zum Rückfluss erhitzt. Innerhalb von 60 Minuten werden 30,2 g Wasser ausgekreist. Die Temperatur des Reaktionsgemisches erhöht sich zum Ende der Reaktion auf 186°C. Das Reaktionsgemisch wird auf Raumtemperatur abgekühlt und mit 162,2 g wässriger Natronlauge (1 Gew.-%) versetzt. Nach Phasentrennung wird die organische Phase zweimal mit insgesamt 911,9 g Wasser gewaschen. Nach zweimaliger Phasentrennung wird die organische Phase (435,9 g) bei einer Sumpftemperatur von 240°C und einem Druck von 100 Pa von Leichtsiedern befreit. Nach Destillation am Dünnschichtverdampfer bei einer Manteltemperatur von 240°C und einem Druck von 100 Pa wird der Ester (321,8 g) in einer Reinheit von 95,6 % isoliert. Dies entspricht einer Ausbeute von 79 % der Theorie.

**Tabelle: Eigenschaften der Esterverbindungen**

| **Ester** | **V₄₀ mm²/s** | **V₁₀₀ mm²/s** | **Pourpoint °C** |
|---|---|---|---|
| Di-(3-(4'-Methylcyclohexyl)butyl)-malonsäureester | 30.9 | 4.9 | -51 |
| Di-(3-(4'-Methylcyclohexyl)butyl)-bemsteinsäureester | 41.1 | 5.8 | -42 |
| Di-(3-(4'-Methylcyclohexyl)butyl)-glutarsäureester | 38.4 | 5.9 | -54 |
| Di-(3-(4'-Methylcyclohexyl)butyl)-adipinsäureester | 38.5 | 6.0 | ≤ -45 |
| Di-(3-(4'-Methylcyclohexyl)butyl)-sebacinsäureester | 50.2 | 7.7 | ≤ -48 |

Bestimmung der kinematischen Viskosität gemäß DIN 51562/ASTM D445.
Bestimmung des Pourpoints gemäß ASTM D97.

Auf Basis des nachwachsenden Rohstoffes Limonen lassen sich über die leicht zugängliche Zwischenstufe Limonanalkohol Dicarbonsäureester gewinnen, die sich trotz eines hohen Molekulargewichts durch hervorragende Viskositäts- und Kälteeigenschaften auszeichnen.

## Patentansprüche

1. Carbonsäureester der allgemeinen Formel 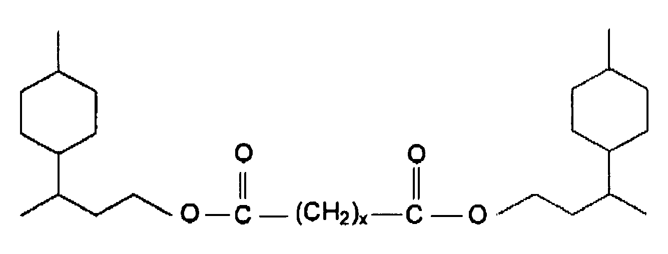 in der x = 1-10, bedeutet.

2. Carbonsäureester nach Anspruch 1, **dadurch gekennzeichnet, dass** x gleich 1, 2, 3, 4, 7, 8 oder 10 bedeutet.

3. Verfahren zur Herstellung der Carbonsäureester gemäß Anspruch 1, durch Umsetzung von 3-(4'-Methylcyclohexyl)butanol mit Dicarbonsäuren der allgemeinen Formel 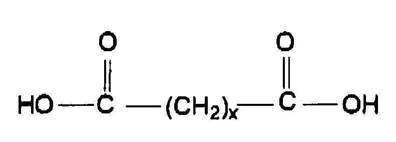 oder deren Anhydriden der allgemeinen Formel 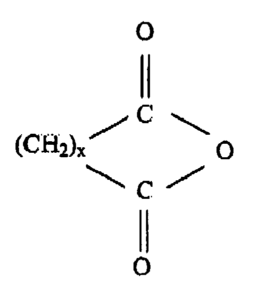 wobei x = 1 - 10 bedeutet, in Gegenwart eines Schleppmittels zur Entfernung des im Verlauf der Umsetzung gebildeten Wassers als azeotropes Gemisch und gegebenenfalls in Gegenwart eines Katalysators, Abtrennung überschüssiger und nicht umgesetzter Ausgangsstoffe, Behandlung mit einem alkalischen Reagenz zur Entfernung acider Bestandteile, gegebenenfalls anschließende Wasserdampfdestillation, und abschließende Trocknung oder fraktionierte Destillation.

4. Verwendung der Carbonsäureester gemäß Anspruch 1 als Schmiermittel oder Weichmacher für thermoplastische Kunststoffe.

5. Schmiermittel auf Basis der Verbindungen gemäß Anspruch 1.

6. Weichmacher auf Basis der Verbindungen gemäß Anspruch 1.

## Claims

1. A carboxylic ester of the general formula 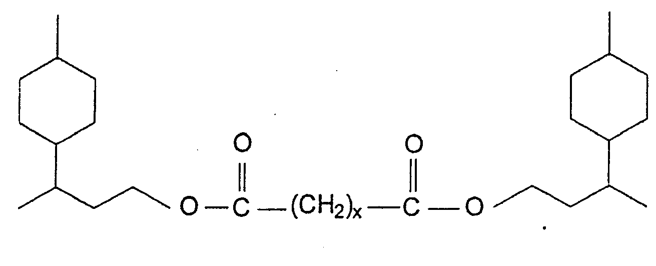 in which x = 1-10.

2. A carboxylic ester as claimed in claim 1, wherein x is 1, 2, 3, 4, 7, 8 or 10.

3. A process for preparing a carboxylic ester as claimed in claim 1, by reacting 3-(4'-methylcyclohexyl)butanol with dicarboxylic acids of the general formula 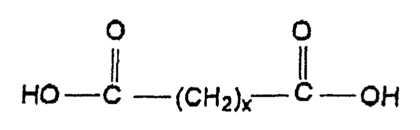 or their anhydrides of the general formula. 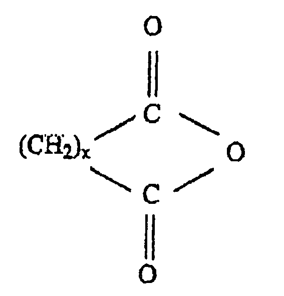 where x = 1-10, in the presence of an azeotroping agent to remove the water formed in the course of the reaction as an azeotropic mixture, and optionally in the presence of a catalyst, removing excess and unconverted starting materials, treating with an alkaline reagent to remove acidic constituents, optionally subsequent steam distillation, and finally drying or fractional distillation.

4. The use of a carboxylic ester as claimed in claim 1 as a lubricant or plasticizer for thermoplastics.

5. A lubricant based on compounds as claimed in claim 1.

6. A plasticizer based on compounds as claimed in claim 1.

## Revendications

1. Esters d'acides carboxyliques de la formule générale 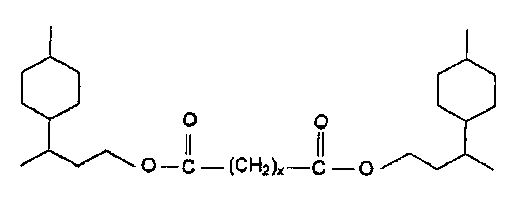 dans laquelle x = 1 à 10.

2. Esters d'acides carboxyliques suivant la revendication 1, **caractérisés en ce que** x vaut 1, 2, 3, 4, 7, 8 ou 10.

3. Procédé de préparation des esters d'acides carboxyliques suivant la revendication 1, par réaction de 3-(4'-méthylcyclohexyl)butanol avec des acides dicarboxyliques de la formule générale 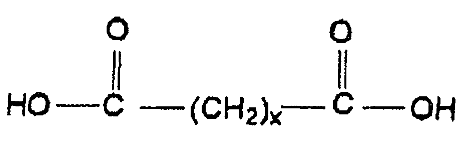 ou leurs anhydrides de la formule générale 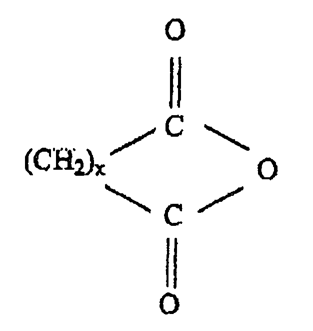 dans laquelle x = 1 à 10, en présence d'un agent d'entraînement pour l'élimination de l'eau formée au cours de la réaction sous forme de mélange azéotrope et le cas échéant en présence d'un catalyseur, élimination de substances de départ en excès et n'ayant pas réagi, traitement par un réactif alcalin pour éliminer des constituants acides, le cas échéant distillation subséquente à la vapeur d'eau, et séchage final ou distillation fractionnée.

4. Utilisation des esters d'acides carboxyliques suivant la revendication 1 en tant que lubrifiants ou plastifiants pour des matières synthétiques thermoplastiques.

5. Lubrifiants à base des composés suivant la revendication 1.

6. Plastifiants à base des composés suivant la revendication 1.
